# EUROPEAN PATENT APPLICATION

(11) **EP 1 852 109 A1**
(43) Date of publication of application: **07.11.2007**
(21) Application number: 06741800.4
(22) Date of filing: 08.05.2006
(51) Int. Cl.: A61K 9/16

(54) **XYLITOL GRANULES CAPABLE OF DIRECTLY BEING PRESSED INTO TABLETS AND PREPARATION PROCESS THEREOF**

(30) Priority: 08.05.2005 CN 200510068255
(71) Applicant: Beijing Jianli Pharmaceutical Co. Ltd., Haidian Beijing 100089 (CN)
(72) Inventor: ZHAO, Li, Beijing 100089 (CN)
(74) Representative: Steimle, Josef
(86) International application number: PCT/CN2006/000905
(87) International publication number: WO 2006/119697

(57) **Abstract**

The present invention provides a xylitol granule directly compressible into tablet mainly comprising xylitol and polyvinyl pyrolidone, and a process for preparing the same, and the granule may comprise sorbitol and hydroxypropylmethyl cellulose etc.

## Description

### FIELD OF THE INVENTION

The present invention relates to the fields of medical care and food, in particular to a process for preparing a xylitol granule directly compressible into tablet and to a xylitol granule thus obtained. The granule comprises xylitol and polyvinyl pyrolidone, and may further comprise sorbitol and hydroxypropylmethyl cellulose.

### BACKGROUND OF THE INVENTION

Xylitol is a sweetener with medically physiological functions and the chemical name thereof is pentitol having a molecular formula of C₅H₁₂O₅. The physical form of xylitol is generally a white crystal powder. The sweetness of xylitol is close to that of sucrose, but xylitol contains lower calorie than sucrose and is absorbed rapidly in human body. As a result, xylitol is not only the substitute of daily sugar for healthy people, but also a nutrient agent and an adjuvant for diabetics. Thereafter, it has been found that xylitol is able to adjust pH value of teethridge and antagonize the fermentation of microbe in oral cavity so as to prevent teeth from demineralization. Therefore, xylitol has special function of preventing decayed tooth. Xylitol has been prepared into various dosage forms, such as tablet, granule, buccal sugar loaf of gum and chewing tablet in USA, Europe, Japan and many other countries. Perfusate and chewing gum prepared by xylitol have been commercial available in China. In the meanwhile xylitol and granule thereof are more and more widely used in food additives, nutritional supplements and medicaments.

Xylitol is highly moisture-absorbable and has a low melting point, thus it is quite difficult to be granulated and further pressed into a tablet. The problem which is commonly present is the fact that xylitol granule has a tendency to adhere to each other so as to subsequently form loaf, the tablet is easily to be adhered to each other and has poor hardness, and also the period of storage is short. Furthermore the appearance of prepared product is not satisfied.

In order to solve the above problems, the inventor has thoroughly studied and obtained a xylitol granule which may be directly orally applied and also directly compressed into tablet. The xylitol granule exhibits high stability and good taste, and is easy to be stored. A tablet which is obtained by directly compressing the granule is advantageous in that homogeneous appearance and color, short disintegration time and the tablet is not easily to be embrittled or broken.

### DISCLOSURE OF THE INVENTION

The present invention provides a xylitol granule directly compressible into tablet, which mainly comprises xylitol in an amount of 90.0∼99.0 wt% and polyvinyl pyrolidone in an amount of 1.0~10.0 wt%, based on total weight of the granule. In a preferred embodiment, the amount of polyvinyl pyrolidone is 1.0~5.0 wt%.

The source of xylitol and polyvinyl pyrolidone is not limited and any commercial available product may be used. And the molecular weight of polyvinyl pyrolidone is also not limited, but is preferable in a range of 25,000-360,000, more preferable in a range of 160,000-360,000. According to the present invention, polyvinyl pyrolidone provided as from K30 to K90 (e.g. the average molecular weight of K30 is in a range of 25,000-40,000, that of K60 is about 160,000 and that of K90 is about 360,000) may be used.

According to the present invention, the xylitol granule may further comprise hydroxypropylmethyl cellulose in an amount of 0∼5.0 wt%, preferable 0.1∼5.0 wt%, based on total weight of the granule. Hydroxypropylmethyl cellulose is a cellulose ether with a wide range of applicable viscosity, preferable viscosity is between 20 and 650 MPa·s.

According to the present invention, the xylitol granule may further comprise sorbitol in an amount of 0∼5.0 wt%, preferable 0.1~5.0 wt%, based on total weight of the granule.

The xylitol granule of the invention may further comprise various accessory ingredients which are commonly known for technician in this field, such as sweetening agent, flavoring agent and stabilizing agent.

The present invention also provides a process for preparing a xylitol granule directly compressible into tablet, which comprises the steps of milling xylitol into a fine powder, and mixing the powder with a gel-like solution of polyvinyl pyrolidone, then granulating the mixture by wet granulation.

In a preferred embodiment, the process of the invention comprises the steps of: (a) milling xylitol into a 60∼90-mesh powder (particle size of about 160~250 µm); (b) dissolving polyvinyl pyrolidone in water and then settling so as to form a gel-like solution; (c) homogeneously mixing the xylitol powder with the gel-like solution of polyvinyl pyrolidone and then granulating by wet granulation; and (d) drying the granule.

In another preferred embodiment, sorbitol which has been milled is mixed with the xylitol powder in said step (a); and an ethanol-containing aqueous solution of hydroxypropylmethyl cellulose is mixed with the gel-like solution of polyvinyl pyrolidone in said step (b).

The present invention further provides a process for preparing a xylitol granule directly compressible into tablet, which comprises the steps of: (a) mixing sorbitol which has been milled with a xylitol powder; (b) dissolving hydroxypropylmethyl cellulose in an ethanol-containing aqueous solution to form a viscose solution; (c) adding the viscose solution of hydroxypropylmethyl cellulose into the powder mixture prepared in said step (a), then granulating and drying; and (d) dissolving polyvinyl pyrolidone in water and settling so as to form a gel-like solution which is then added into the granule prepared in said step (c), then granulating and drying again.

In the process according to the present invention, the gel-like solution of polyvinyl pyrolidone is typically settled for 20-40 minutes. And the solvent of hydroxypropylmethyl cellulose is typically an aqueous solution of ethanol in a concentration of 2-10% (v/v). A viscous liquid is obtained after dissolving hydroxypropylmethyl cellulose.

Polyvinyl pyrolidone is a favorable binder and may form a complex compound together with various substances, especially the compound containing amino group or hydroxyl, so that the stability of parent compound such as xylitol is significantly increased without altering physiologically active effect thereof. Polyvinyl pyrolidone has not only excellent adhesive effect, but also has a function of rapid disintegration.

The xylitol granule according to the present invention may be used as additive ingredient in food and/or medicament, such as candy, cake, beverage, health care food or medicament. The xylitol granule according to the present invention may be directly pressed into tablet and the appearance of resulted tablet is smooth and glossy without fissured or cracked surface, and the shape of section is homogeneous.

### EMBODIMENTS OF THE INVENTION

The invention will be described in detail below with reference to the examples, which are not intended to limit the scope of the invention. "%" used in the examples refers to weight percentage, unless other special indication is present.

### Example 1

95.0 g of xylitol was milled and sieved through a 80∼90-mesh sieve. 5.0 g of polyvinyl pyrolidone (K30, Dexiang Medical Technology Co. Ltd., Shanghai, China) was dissolved in water to form a gel-like solution which was then added into the xylitol powder and mixed homogeneously. The mixture was granulated by using a 16-mesh sieve and screened by using a 20-mesh sieve. The resulted granules were dried below 80°C and xylitol granules directly compressible into tablet were prepared.

### Example 2

99.0 g of xylitol was milled and sieved through a 100-mesh sieve. 1.0 g of polyvinyl pyrolidone (K60, Dexiang Medical Technology Co. Ltd. Shanghai, China) was dissolved in water and settled for 20 minutes. The solution of polyvinyl pyrolidone was added into the xylitol powder and mixed homogeneously. The mixture was granulated by using a 16-mesh sieve. The resulted granules were dried at about 80°C and xylitol granules directly compressible into tablet were prepared.

### Example 3

94.0 g of xylitol was milled and sieved through a 100-mesh sieve. 6.0 g of polyvinyl pyrolidone (K60, Dexiang Medical Technology Co. Ltd. Shanghai, China) was dissolved in water to form a gel-like solution which was then added into the xylitol powder and mixed homogeneously. The mixture was granulated and the resulted granules were dried at about 80°C, and xylitol granules directly compressible into tablet were prepared.

### Example 4

92.5 g of xylitol was milled and sieved through a 90-mesh sieve. 1.0 g of hydroxylpropylmethyl cellulose was dissolved in an aqueous solution with an ethanol concentration of 8% (v/v). 6.5 g of polyvinyl pyrolidone (K90, Dexiang Medical Technology Co. Ltd. Shanghai, China) was dissolved in water and settled for 30 minutes to form a gel-like solution. The solution of polyvinyl pyrolidone was mixed with the solution of hydroxypropylmethyl cellulose, and the mixed solution was added into the xylitol powder with stirring and mixed homogeneously. The mixture was granulated by using a 16-mesh sieve. The resulted granules were dried at about 80°C and xylitol granules directly compressible into tablet were prepared.

### Example 5

92.5 g of xylitol was milled and sieved through a 80-mesh sieve. 7.5 g of polyvinyl pyrolidone (K60, Dexiang Medical Technology Co. Ltd. Shanghai, China) was dissolved in water and settled for 20 minutes. The solution of polyvinyl pyrolidone was added into the xylitol powder with stirring and then mixed for 30 minutes to prepare a damp mass. The damp mass was granulated by using a 20-mesh sieve. The resulted granules were dried at about 80 °C and xylitol granules directly compressible into tablet were prepared.

### Example 6

90.0 g of xylitol was milled and sieved through a 60-mesh sieve. 1.5 g of hydroxypropylmethyl cellulose was dissolved in an aqueous solution with an ethanol concentration of 5% (v/v) to form a transparent viscose solution. The solution of hydroxypropylmethyl cellulose was added into the xylitol powder and mixed homogeneously. The mixture was granulated by using a 16-mesh sieve. The resulted granules were dried at 80~85°C. The granules were milled after being cooled so as to sieve through a 90-mesh sieve.

8.5 g of polyvinyl pyrolidone (K60, Dexiang Medical Technology Co. Ltd. Shanghai, China) was dissolved in water to form a gel-like solution. The solution was then added into the powder mixture of xylitol and hydroxypropylmethyl cellulose as described above and mixed homogeneously. The mixture was granulated by using a 20-mesh sieve. The resulted granules were dried at about 80°C for 2 hours and then screened by using a 24-mesh sieve. Xylitol granules directly compressible into tablet were prepared.

### Example 7

Xylitol granules directly compressible into tablet were prepared as described in Example 5, except that 91.0 g of xylitol and 9.0 g of polyvinyl pyrolidone (K90, Dexiang Medical Technology Co. Ltd. Shanghai, China) were used therein.

### Example 8

Xylitol granules directly compressible into tablet were prepared as described in Example 5, except that 90.0 g of xylitol and 10.0 g of polyvinyl pyrolidone (K90) were used therein.

### Example 9

90.0 g of xylitol and 1.5 g of sorbitol were milled respectively and sieved through a 100-mesh sieve, subsequently mixed homogeneously. 8.5 g of polyvinyl pyrolidone (K60) was dissolved in water and settled for 20 minutes. The solution of polyvinyl pyrolidone was added into the mixture of xylitol and sorbitol, then mixed for 30 minutes and granulated by using a 16-mesh sieve. The resulted granules were dried at 80~85°C for 1.5~2 hours, and then screened by using a 20-mesh sieve. Thereafter they were dried for 0.5 hour and xylitol granules directly compressible into tablet were prepared.

### Example 10

95.0 g of xylitol was milled and sieved through a 100-mesh sieve; 2.5 g of sorbitol was milled and sieved through a 100-mesh sieve, both of which were mixed. 1.5 g of hydroxypropylmethyl cellulose was dissolved in an aqueous solution with an ethanol concentration of 5% (v/v), and 1.0 g of polyvinyl pyrolidone (K60) was dissolved in water to form a gel-like solution. Both solutions were mixed and added into the powder mixture of xylitol and sorbitol as described above. They were mixed and granulated, and then screened by using a 18~20-mesh sieve. The resulted granules were dried below 80°C for 1.5 hours and xylitol granules directly compressible into tablet were prepared.

### Example 11

92.0 g of xylitol and 0.5 g of sorbitol were milled respectively and sieved through a 100-mesh sieve, subsequently mixed homogeneously. 1.0 g of hydroxypropylmethyl cellulose was dissolved in an aqueous solution with an ethanol concentration of 2% (v/v); and 6.5 g of polyvinyl pyrolidone (K60) was dissolved in water to form a gel-like solution which was then added into the powder mixture of xylitol and sorbitol as described above. The solution of hydroxypropylmethyl cellulose was added after 10-minutes stirring and then the mixture was granulated by using a 20-mesh sieve after 20-minutes mixing. The resulted granules were dried at about 80°C for about 2 hours and subsequently screened by using a 24-mesh sieve. Xylitol granules directly compressible into tablet were prepared.

### Example 12

The prepared granules in above Examples 1-11 were respectively pressed into tablet and 10 tablets (0.8 g for each tablet) were prepared for each Example. It is observed visually that the surface of each tablet was smooth without apparent protrusion. The surface was not fissured or cracked and the section after being broken off was homogeneous in texture.

### Example 13: The effect of molecular weight (viscosity) of polyvinyl pyrolidone

According to the procedure in Example 1, polyvinyl pyrolidone with various viscosities (K15, K20, K30, K40, K60, K90, K100, and K120) was respectively granulated with xylitol. Subsequently the prepared granules were directly pressed into tablet. According to the standard in CP (Chinese Pharmacopoeia), edition 2005, volumn II, appendix X_{A}, the comparative test for disintegration time of tablet was carried out, and the results were shown in the table below.

**Table 1**

| | K15 | K20 | K30 | K40 | K60 | K90 | K100 | K120 |
|---|---|---|---|---|---|---|---|---|
| Disintegration time (min) | 2.6 | 2.6 | 2.8 | 3.0 | 5.0 | 6.0 | 15.0 | >20.0 |

## Claims

1. A xylitol granule directly compressible into tablet, which mainly comprises xylitol in an amount of 90.0~99.0 wt% and polyvinyl pyrolidone in an amount of 1.0~10.0 wt%, based on total weight of the granule.

2. The xylitol granule according to claim 1, which comprises polyvinyl pyrolidone in an amount of 1.0∼5.0 wt%, based on total weight of the granule.

3. The xylitol granule according to claim 1, wherein said polyvinyl pyrolidone is K30 to K90.

4. The xylitol granule according to claim 1, wherein said granule further comprises hydroxypropylmethyl cellulose.

5. The xylitol granule according to claim 4, wherein said granule comprises hydroxypropylmethyl cellulose in an amount of 0∼5.0 wt%, and the viscosity of said hydroxypropylmethyl cellulose is between 20 and 650 MPa.s.

6. The xylitol granule according to claim 5, wherein said granule comprises hydroxypropylmethyl cellulose in an amount of 0.1~5.0 wt%.

7. The xylitol granule according to claim 1, wherein said granule further comprises sorbitol.

8. The xylitol granule according to claim 7, wherein said granule comprises sorbitol in an amount of 0∼5.0 wt%.

9. The xylitol granule according to claim 8, wherein said granule comprises sorbitol in an amount of 0.1~5.0 wt%.

10. The xylitol granule according to claim 1, wherein said polyvinyl pyrolidone has a molecular weight in a range of 25,000-360,000.

11. A process for preparing a xylitol granule directly compressible into tablet, which comprises the steps of milling xylitol into a fine powder, and mixing the powder with a gel-like solution of polyvinyl pyrolidone, then granulating the mixture by wet granulation.

12. The process according to claim 11, which comprises the steps of:
- (a) milling xylitol into a 60∼90-mesh powder (particle size of about 160~250 µm);
- (b) dissolving polyvinyl pyrolidone in water and then settling so as to form a gel-like solution;
- (c) homogeneously mixing the xylitol powder with the gel-like solution of polyvinyl pyrolidone and then granulating by wet granulation; and
- (d) drying the granule.

13. The process according to claim 12, wherein sorbitol which has been milled is mixed with the xylitol powder in said step (a); and an ethanol-containing aqueous solution of hydroxypropylmethyl cellulose is mixed with the gel-like solution of polyvinyl pyrolidone in said step (b).

14. A process for preparing a xylitol granule directly compressible into tablet, which comprises the steps of:
- (a) mixing sorbitol which has been milled with a xylitol powder;
- (b) dissolving hydroxypropylmethyl cellulose in an ethanol-containing aqueous solution to form a viscose solution;
- (c) adding the viscose solution of hydroxypropylmethyl cellulose into the powder mixture prepared in said step (a), then granulating and drying; and
- (d) dissolving polyvinyl pyrolidone in water and settling so as to form a gel-like solution which is then added into the granule prepared in said step (c), then granulating and drying again.

15. The process according to any one of claims 12-14, wherein said drying is carried out below 80°C.

16. Use of the xylitol granule according to any one of claims 1-10 as additive ingredients in food and/or medicament.
